# EUROPÄISCHE PATENTSCHRIFT

(11) **EP 1 207 844 B1**
(45) Veröffentlichungstag und Bekanntmachung des Hinweises auf die Patenterteilung: **26.04.2006**
(21) Anmeldenummer: 00958484.8
(22) Anmeldetag: 22.08.2000
(51) Int. Cl.: A61Q 5/06, A61Q 5/10, A61K 8/41

(54) **HAARFÄRBEVERFAHREN**
METHOD FOR COLOURING HAIR
PROCEDE POUR LA COLORATION DES CHEVEUX

(30) Priorität: 31.08.1999 DE 19941450
(43) Veröffentlichungstag der Anmeldung: 29.05.2002
(73) Patentinhaber: Henkel Kommanditgesellschaft auf Aktien, 40589 Düsseldorf (DE)
(72) Erfinder: AKRAM, Mustafa, D-22457 Hamburg (DE); HAUBOLD, Rolf-Werner, D-22763 Hamburg (DE)
(86) Internationale Anmeldenummer: PCT/EP2000/008175
(87) Internationale Veröffentlichungsnummer: WO 2001/015662

(56) Entgegenhaltungen:
- US-A- 4 425 132
- US-A- 5 073 174

## Beschreibung

Die vorliegende Erfindung betrifft ein zweistufiges Verfahren zum Färben keratinischer Fasern sowie die Verwendung eines speziellen Vorbehandlungsmittels in einem derartigen Verfahren.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel wie beispielsweise Wasserstoffperoxid-Lösungen. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Färbemittel auf der Basis direktziehender Farbstoffe kommen ohne Oxidationsmittel aus und können bei pH-Werten im Bereich des Neutralpunktes formuliert werden. Ein wesentlicher Nachteil der Färbemittel auf Basis direktziehender Farbstoffe ist die geringe Waschechtheit der erzielten Färbungen. Das Aufziehvermögen der Farbstoffmoleküle auf das Haar sowie der Glanz der gefärbten Haaren können in vielen Fällen ebenfalls nicht voll befriedigen.

Dennoch vermögen diese Färbemethoden des Standes der Technik noch nicht vollständig zu überzeugen. Häufig entsteht bei Färbungen, insbesondere bei den sogenannten Modenuancen im Rot-, Kupfer- und Goldbereich, ein ungleichmäßiger Farbeindruck, und die Haare weisen in den Spitzen einen Verlust von Brillanz auf. Diese Unregelmäßigkeiten der Färbung resultieren aus dem unterschiedlichen Pflegezustand der Haare in den frisch nachgewachsenen Teilen und den gegebenenfalls bereits durch frühere Behandlungen oder Umwelteinflüsse poröseren Spitzen. Daher war es die Aufgabe der vorliegenden Erfindung, ein Färbeverfahren für keratinische Fasern bereitzustellen, das die oben genannten Nachteile nicht aufweist und zu Färbungen mit hervorragenden Echtheitseigenschaften führt.

Es wurde nun überraschender Weise gefunden, daß sehr gleichmäßige Färbungen, auch im Bereich modischer Rot-, Kupfer- und Goldnuancen, mit hervorragenden Echtheitseigenschaften erzielt werden können, wenn die Fasern in einem ersten Schritt mit einem Vorbehandlungsmittel, enthaltend direktziehende Farbstoffe sowie einen faserstrukturverbessernden Wirkstoff, behandelt werden und in einem zweiten Schritt einer herkömmlichen Färbung mit mindestens einem synthetischen Farbstoff(vorprodukt) unterzogen werden.

Zweistufige Verfahren zum Färben keratinischer Fasern sind bereits seit langem bekannt. So offenbart beispielsweise die WO-90/01922-A1 ein Haarfärbeverfahren, bei dem die Haare in einem ersten Schritt mit einem Mittel, enthaltend einen direktziehenden Farbstoff, und in einem zweiten Schritt mit einem Mittel, enthaltend Naturfarbstoffe, behandelt werden. Dieser Schrift sind aber keinerlei Hinweise auf die Vorzüge des hier beanspruchten Verfahrens zu entnehmen. Häufig waren derartige Verfahren aber sehr komplex und konnten nur vom Spezialisten durchgeführt werden. Ferner konnten die mit diesem Verfahren erhaltenen Färbungen noch nicht alle Wünsche hinsichtlich der Egalisierung und der Echtheitseigenschaften erfüllen.

Ein erster Gegenstand der vorliegenden Erfindung ist daher ein zweistufiges Verfahren zur Färbung keratinischer Fasern, bei dem in einem ersten Schritt die Fasern mit einem Vorbehandlungsmittel, enthaltend mindestens einen direktziehenden Farbstoff sowie einen faserstrukturverbessernden Wirkstoff, und in einem zweiten Schritt mit einem herkömmlichen Färbemittel mit mindestens einem synthetischen Farbstoff(vorprodukt) behandelt werden.

Unter Keratinfasern sind dabei Pelze, Wolle, Federn und insbesondere menschliche Haare zu verstehen.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Basic Yellow 57, Acid Yellow 1 (CI 10316), Disperse Orange 3, HC Red 3, HC Red BN, Basic Red 2, Basic Red 76, Acid Red 87 (CI 45380), HC Blue 2, HC Blue 12, Disperse Blue 3, Basic Blue 7, Basic Blue 99, FD&C Green 3 (CI 42053), D&C Green No. 8 (CI 59040), HC Violet 1, Basic Violet 14, Disperse Violet 1, Disperse Violet 4, Disperse Black 9, Acid Black 52, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, Natriumpikramat, 2-Amino-6-chloro-4-nitrophenol, 4-Ethylamino-3-nitrobenzoesäure und 2-Chloro-6-ethylamino-1-hydroxy-4-nitrobenzol.

Obwohl das Vorbehandlungsmittel prinzipiell alle direktziehenden Farbstoffe enthalten kann, haben sich, insbesondere wenn die Färbung der zweiten Stufe oxidativ erfolgt, Nitrobenzolderivate, basische direktziehende Farbstoffe und Triphenylmethanderivate als besonders geeignet erwiesen. Besonders bevorzugte Farbstoffe sind im Rahmen der vorliegenden Erfindung 3-Nitro-4-aminophenol, 4-(3'-Hydroxypropylamino)-3-nitrophenol (HC Red BN), 1,4-Bis-(β-hydroxyethyl)-amino-2-nitrobenzol, 1-(β-Hydroxyethylamino)-2-nitro-4-aminobenzol (HC Red No. 3), 1-(β-Hydroxyethylamino)-2-nitrobenzol (HC Yellow No. 2), 4-(β-Hydroxyethylamino)-3-nitrophenol, HC Blue 2, Basic Blue 99, Basic Red 76, Basic Brown 16 und Basic Brown 17.

Die erfindungsgemäßen Vorbehandlungsmittel enthalten die direktziehenden Farbstoffe bevorzugt in einer Menge von 0,001 bis 20 Gew.-%, bevorzugt zwischen 0,005 und 5 Gew.-%, ganz besonders bevorzugt zwischen 0,01 und 1 Gew.-%, bezogen auf das gesamte Vorbehandlungsmittel.

Das erfindungsgemäße Vorbehandlungsmittel enthält die direktziehenden Farbstoffe in einem geeigneten wasserhaltigen Träger. Zusätzlich können die Vorbehandlungsmittel weitere organische Lösemittel, wie beispielsweise Methoxybutanol, Benzylalkohol, Isopropylalkohol, Ethyldiglykol oder 1,2-Propylenglykol, enthalten. Bevorzugt sind dabei alle wasserlöslichen organischen Lösemittel.

Erfindungsgemäße Träger sind weiterhin Cremes, Emulsionen, Gele oder auch tensidhaltige schäumende Lösungen, z.B. Shampoos, Schaumaerosole oder andere Zubereitungen, die für die Anwendung auf dem Haar geeignet sind. Obwohl einer Formulierung des Vorbehandlungsmittels in einer der genannten Formen nichts entgegensteht, hat es sich im Rahmen der vorliegenden Erfindung als besonders vorteilhaft hinsichtlich der erzielbaren Gleichmäßigkeit der Färbung und der einfacheren Handhabbarkeit erwiesen, wenn das Vorbehandlungsmittel als Spray formuliert wird.

Erfindungsgemäß besonders bevorzugt ist eine Formulierung als Treibmittel-freies Pumpspray. Gleichwohl können diese Zubereitungen auch als treibmittelhaltige Mittel formuliert werden. Prinzipiell können dabei als Treibmittel alle auf dem Gebiet der Kosmetik verwendeten, verflüssigbaren Gase sowie Luft eingesetzt werden. Dazu zählen beispielsweise niedere Alkane, Dimethylether sowie Fluorchlor- und Fluorkohlenwasserstoffe. Bevorzugt werden das oder die Treibmittel so gewählt, daß die erfindungsgemäßen Zubereitungen auch über längere Zeiten bei den für Kosmetika üblichen Lagerbedingungen stabile homogene Gemische bilden. Bevorzugte Treibmittel sind niedere Alkane, insbesondere Propan, Butan, Isobutan, sowie Mischungen dieser Alkane.

Die erfindungsgemäßen Zubereitungen enthalten die Treibmittel bevorzugt in Mengen von 1 - 20 Gew.-%, insbesondere in Mengen von 2 - 10 Gew.-%, bezogen auf die gesamte Zubereitung.

Das Vorbehandlungsmittel enthält neben den direktziehenden Farbstoffen einen faserstrukturverbessernden Wirkstoff. Beispiele für faserstrukturverbessernde Wirkstoffe stellen Panthenol und seine physiologisch verträglichen Derivate dar. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Panthenol ist innerhalb dieser Gruppe bevorzugt.

Eine weitere geeignete Gruppe von faserstrukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäße Verwendung geeignet sind die Extrakte aus Mandel, Aloe Vera. Kokosnuß, Mango, Aprikose, Limone, Weizen, Kiwi, Melone und grünem Tee.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenelykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als faserstrukturverbessemde Wirkstoffe bevorzugt sind Honigextrakte. Diese Extrakte werden in analoger. Weise zu den Pflanzenextrakten gewonnen und enthalten üblicherweise 1 - 10 Gew.-%, insbesondere 3 - 5 Gew.-%, Aktivsubstanz. Wasser/Propylenglykol-Mischungen können auch hier bevorzugte Extraktionsmittel sein.

Weitere faserstrukturverbessemde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quatemisierte Proteinhydrolysate. Ebenfalls faserstrukturverbessemde Wirkstoffe sind Mono-, Di- und Oligosaccharide wie beispielsweise Glucose, Galactose, Fructose, Fruchtzucker und Lactose.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Serin ist ein besonders bevorzugter faserstrukturverbessernder Wirkstoff.

Die erfindungsgemäßen Vorbehandlungsmittel können weiterhin alle für solche Zubereitungen bekannten Wirk-, Zusatz- und Hilfsstoffe enthalten. In vielen Fällen enthalten die Vorbehandlungsmittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46 und Dehyquart® L-80. Die Esterquats sind erfindungsgemäß besonders bevorzugte Tenside.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure.
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Antioxidantien.

Die Einwirkzeit des Vorbehandlungsmittels unterliegt keinerlei Beschränkungen. Es hat sich aber als vorteilhaft erwiesen, wenn die Einwirkzeit zwischen 1 und 30 Minuten, insbesondere zwischen 1 und 10 Minuten, ganz besonders bevorzugt zwischen 3 und 5 liegt. Auch wenn prinzipiell vor der herkömmlichen Haarfärbung im zweiten Schritt keine Zwischenbehandlung erforderlich ist, hat es sich als vorteilhaft erwiesen, wenn nach der Einwirkzeit überschüssige Flüssigkeit vor der Anwendung des zweiten Haarfärbemittels abgetupft, abgestreift oder abgetrocknet wird.

Die erfindungsgemäßen Haarbehandlungsmittel werden bevorzugt derart formuliert, daß sie einen pH-Wert zwischen 3 und 9, bevorzugt zwischen 4 und 8, ganz besonders bevorzugt zwischen 4 und 5 aufweisen. Zur Einstellung des pH-Wertes können alle in der Kosmetik zu diesem Zwecke eingesetzten Säuren und Alkalisierungsmittel zum Einsatz kommen.

Der Begriff herkömmliche Färbung mit mindestens einem synthetischen Farbstoff(vorprodukt) im zweiten Schritt des erfindungsgemäßen Verfahrens umfaßt dabei alle dem Fachmann bekannten Verfahren, bei denen auf das Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Die Zusammensetzung des herkömmlichen Färbemittels unterliegt keinen prinzipiellen Einschränkungen.
Als synthetische Farbstoff(vorprodukt)e können
- Oxidationsfarbstoffvorprodukte vom Entwickler- und Kuppler-Typ,
- synthetische direktziehende Farbstoffe und
- Vorstufen naturanaloger Farbstoffe, wie Indol- und Indolin-Derivate,
sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazoldcrivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Die Entwicklerkomponenten p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol und 2,4,5,6-Tetraaminopyrimidin haben sich als besonders bevorzugt, insbesondere zur Erzielung der Modenuancen, erwiesen.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2`-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorein, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Antino-3,4-methylendioxybeazol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Di-(β-hydroxyethylamino)-toluol und 2,6-Dihydroxy-3,4-dimethylpyridin.

Ganz besonders bevorzugte Kupplerkomponenten, insbesondere zur Erzielung der modemen Nuancen in Rotbereich sind 2-Chlor-6-methyl-3-aminophenol, 5-Amino-2-methylphenol, 2-Amino-3-hydroxypyridin, 2,6-Di-(β-hydroxyethylamino)-toluol, 2-Methylresorcin und 1-Naphthol.

Die Entwickler-/Kupplerkombination 2,4,5,6-Tetraaminopyrimidin und 2-Methylresorcin ist besonders bevorzugt zur Erzielung rötlicher Nuancen.

Hinsichtlich der erfindungsgemäß geeigneten direktziehenden Farbstoffe sei an dieser Stelle auf die obigen Ausführungen verwiesen.

Zur weiteren Nuancierung können neben dem synthetischen Farbstoff(vorprodukt) auch der Natur vorkommende direktziehende Farbstoffe, wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel, eingesetzt werden.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfarbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dennatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Sowohl die Oxidationsfarbstoffvorprodukte als auch die direktziehenden Farbstoffe sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (Ia), 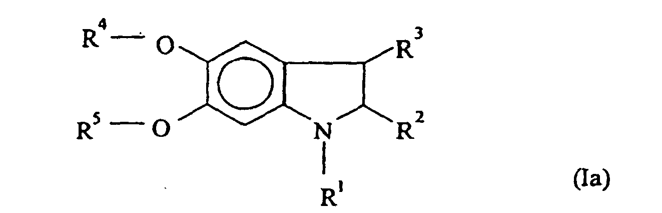 in der unabhängig voneinander
R¹ steht für Wasserstoff, eine C₁-C₄-Alkylgruppe oder eine C₁-C₄-Hydroxy-alkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 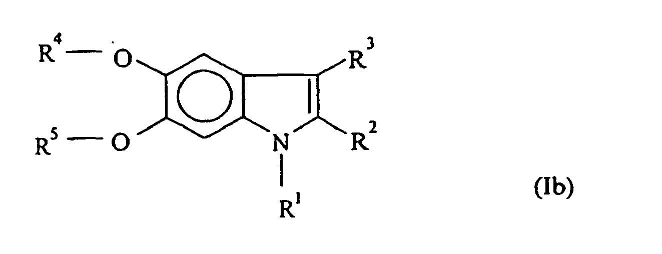 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxylndol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropatiolamin, 2-Amino-2-methylpropanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel.

So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten-wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Ein weiterer Gegenstand der vorliegenden Erfindung ist die Verwendung eines Vorbehandlungsmittels, enthaltend mindestens einen direktziehenden Farbstoff sowie einen faserstrukturverbessernden Wirkstoff, in Rahmen einer Haarfärbung zur Erzielung einer gleichmäßigen Färbung.

In einer bevorzugten Ausführungsform dieses Gegenstandes der Erfindung ist das Vorbetiandlungsmittel als Spray konfektioniert. Zur weiteren Ausgestaltung sei auf die obigen Ausführungen verwiesen.

Die folgenden Beispiele sollen den Gegenstand der vorliegenden Erfindung näher erläutern.

### Beispiele

Alle Mengenangaben in den folgenden Beispielen sind, soweit nichts anderes vermerkt ist, Gewichtsteile.

### Beispiel 1

Bei einer Versuchsperson mit dunkelblonden Haaren mit stark porösen Spitzen wurde die Frisur durch einen Mittelscheitel in eine linke und eine rechte Hälfte geteilt.
Die linke Seite wurde wie folgt behandelt:
Die porösen Haarspitzen wurden mit ca. 15g der Formulierung A1 eingesprüht. Nach einer Einwirkzeit von 8 Minuten bei 25°C wurde die gesamte Haarlänge mit einer Mischung aus 30g der Formulierung B1 und 30g einer 6%igen wäßrigen Wasserstoffperoxidlösung behandelt. Nach einer Einwirkzeit von 30 Minuten bei 25 °C wurden die Haare mit Wasser ausgespült, nachshampooniert und mit dem Fön getrocknet.
Die rechte Seite wurde wie folgt behandelt:
Die gesamte Haarlänge wurde mit einer Mischung aus 30 g der Formulierung B und 30 g einer 6%igen wäßrigen Wasserstoffperoxidlösung behandelt. Nach einer Einwirkzeit von 30 Minuten bei 25°C wurden die Haare mit Wasser ausgespült, nachshampooniert und mit dem Fön getrocknet.

### Ergebnis :

Die linke Seite zeigte eine von dem Ansatz bis zur Spitze gleichmäßige und intensive Rotfärbung.
Die rechte Seite zeigte eine weniger gleichmäßige und weniger intensive Rotfärbung.

### Haltbarkeitsversuch:

Die Kopfhaare wurden beidseitig 12 Tage lang täglich mit einem handelsüblichen Shampoo gewaschen. Das Ergebnis zeigte, daß der Farbabtrag auf der rechten Seite stärker ist als auf der linken Seite.

### Formulierung A1

| | |
|---|---|
| Natrosol® 250 HR¹ | 0,2 |
| Dehyquart® L-80² | 0,3 |
| Panthenol | 0,1 |
| p-Hydroxybenzoesäurernethylester | 0,03 |
| p-Hydroxybenzoesäurepropylester | 0,03 |
| Methoxybutanol | 2,0 |
| 4-Hydroxypropylamino-3-nitrophenol | 0,2 |
| Basic Red 76 | 0,2 |
| Wasser | ad 100 |

| | |
|---|---|
| ¹ Hydroxyethylcellulose (Fa. Hercules) | |
| ² Bis(Cocoyl)ethyl-hydroxyethyl-methyl-ammonium-Methosulfat (INCI-Bezeichnung: Dicocoylethyl hydroxyethylmonium methosulfate, propylene glycol; Henkel) | |

### Formulierung B1

| | |
|---|---|
| Ammoniumcarbopol-Lösung, 1%ig in Wasser³ | 17,25 |
| Ammoniumrohagitlösung, 6%ig in Wasser⁴ | 5,25 |
| Oleth-7⁵ | 5,70 |
| Kaliumoleinseife,12,5%ig in Wasser | 3,45 |
| Plantaren®2000⁶ | 0,53 |
| Titandioxid Anatas, Typ AS 05 | 0,48 |
| Cetiol® V⁷ | 3,45 |
| Cetylalkohol | 16,80 |
| Glycerinmonostearat NSE⁸ | 2,85 |
| Phospholipid EFA⁹ | 0,85 |
| Tetrasodium EDTA | 0,46 |
| Kieselsäure, hochdisperses, pyrogen | 0,11 |
| p-Toluylendiamin | 0,1 |
| 2,4,5,6- Tetraaminopyrimidin | 1,5 |
| 2-Methylresorcin | 0,8 |
| 2-Amino-3-hydroxypyridin | 0,05 |
| HC Red BN | 0,8 |
| HC Red 3 | 0,1 |
| Methoxybutanol | 1,43 |
| Ammoniak, 25%ig im Wasser | ad pH 10,0 |
| Ascorbinsäure | 0,1 |
| Cetyltrimethylammoniumbromid | 0,5 |
| Parfüm | 0,43 |
| Wasser | ad 100,00 |

| | |
|---|---|
| ³ Lösung eines Ammoniumsalzes eines Methacrylsäure-Methylacrylat-Copolymeren (INCI-Bezeichnung: Ammonium Polyacrylate) (Röhm GmbH) | |
| ⁴ Lösung eines Ammoniumsalzes eines Acrylsäure-Polymeren (INCI-Bezeichnung: Ammonium Acrylates Copolymer) (Goodrich) | |
| ⁵ Oleylalkohol mit 7 EO-Einheiten (Henkel) | |
| ⁶ C₈₋₁₆-Alkyl-1,4-Polyglucosid (ca. 51% Aktivsubstanz; INCI-Bezeichnung: Decyl Glucoside) (Henkel) | |
| ⁷ Ölsäuredecylester (INCI-Bezeichnung: Decyl Oleate) (Henkel) | |
| ⁸ (INCI-Bezeichnung: Glyceryl Strearate) (Oleofina) | |
| ⁹ ca. 30% Aktivsubstanz; INCI-Bezeichnung: Linoleamidopropyl PG-Dimonium Chloride Phosphate (Mona) | |

### Beispiel 2

Bei einer Versuchsperson mit mittelblonden Haaren mit stark porösen Spitzen wurde die Frisur durch einen Mittelscheitel in eine linke und eine rechte Hälfte geteilt.

### Die linke Seite wurde wie folgt behandelt:

Die porösen Haarspitzen wurden mit ca. 10 g der Formulierung A2 eingesprüht. Nach einer Einwirkzeit von 7 Minuten bei 25°C wurde die gesamte Haarlänge mit einer Mischung aus 30g der Formulierung B2 und 30g einer 6%igen wäßrigen Wasserstoffperoxidlösung behandelt. Nach einer Einwirkzeit von 30 Minuten bei 25 °C wurden die Haare mit Wasser ausgespült, nachshampooniert und mit dem Fön getrocknet. Die rechte Seite wurde wie folgt behandelt:
Die gesamte Haarlänge wurde mit einer Mischung aus 30g der Formulierung B2 und 30g einer 6%igen wäßrigen Wasserstoffperoxidlösung behandelt. Nach einer Einwirkzeit von 30 Minuten bei 25°C wurden die Haare mit Wasser ausgespült, nachshampooniert und mit dem Fön getrocknet.

### Ergebnis :

Die linke Seite zeigte eine von dem Ansatz bis zur Spitze gleichmäßige und intensive Kupferfärbung.
Die rechte Seite zeigte eine weniger gleichmäßige und weniger intensive Kupferfärbung.

### Haltbarkeitsversuch:

Die Kopfhaare wurden beidseitig 12 Tage lang täglich mit einem handelsüblichen Shampoo gewaschen. Das Ergebnis zeigte, daß der Farbabtrag auf der rechten Seite stärker ist als auf der linken Seite.

### Formulierung A2

| | |
|---|---|
| Natrosol® 250 HR | 0,2 |
| Dehyquart® L-80 | 0,3 |
| Panthenol | 0,1 |
| p-Hydroxybenzoesäurernethylester | 0,03 |
| p-Hydroxybenzoesäurepropylester | 0,03 |
| Methoxybutanol | 2, |
| 3-Nitro-4-aminophenol | 0,2 |
| Wasser | ad 100 |

### Formulierung B2

| | |
|---|---|
| Ammoniumcarbopol-Lösung, 1%ig in Wasser | 17,25 |
| Ammoniumrohagitlösung, 6%ig in Waser | 5,25 |
| Oleth-7 | 5,70 |
| Kaliumoleinseife,12,5%ig in Wasser | 3,45 |
| Plantaren®2000 | 0,53 |
| Titandioxid Anatas, Typ AS 05 | 0,48 |
| Cetiol® V | 3,45 |
| Cetylalkohol | 16,80 |
| Glycerinmonostearat NSE | 2,85 |
| Phospholipid EFA | 0,85 |
| Tetrasodium EDTA | 0,46 |
| Kieselsäure, hochdisperses, pyrogen | 0,11 |
| p-Toluylendiamin | 0.4 |
| 2-Amino-3-hydroxypyridin | 0,11 |
| 3-Methyl-4-aminophenol | 0,3 |
| Resorcin | 0,06 |
| 2-Methylresorcin | 0,04 |
| 2-Hydroxy-4-aminotoluol | 0,2 |
| Methoxybutanol | 1,43 |
| Ammoniak, 25%ig im Wasser | ad pH 10,0 |
| Ascorbinsäure | 0,1 |
| Cetyltrimethylammoniumbromid | 0,5 |
| Parfüm | 0,43 |
| Wasser | ad 100,00 |

### Beispiel 3

Bei einer Versuchsperson mit hellblonden Haaren mit stark porösen Spitzen wurde die Frisur durch einen Mittelscheitel in eine linke und eine rechte Hälfte geteilt.

### Die linke Seite wurde wie folgt behandelt:

Die porösen Haarspitzen wurden mit ca. 7g der Formulierung A3 eingesprüht. Nach einer Einwirkzeit von 5 Minuten wurde die gesamte Haarlänge mit einer Mischung aus 30g der Formulierung B3 und 30g einer 6%igen wäßrigen Wasserstoffperoxidlösung behandelt. Nach einer Einwirkzeit von 30 Minuten bei 25 °C wurden die Haare mit Wasser ausgespült, nachshampooniert und mit dem Fön getrocknet.
Die rechte Seite wurde wie folgt behandelt:
Die gesamte Haarlänge wurde mit einer Mischung aus 30g der Formulierung B3 und 30g einer 6%igen wäßrigen Wasserstoffperoxidlösung behandelt. Nach einer Einwirkzeit von 30 Minuten bei 25°C wurden die Haare mit Wasser ausgespült, nachshampooniert und mit dem Fön getrocknet.

### Ergebnis :

Die linke Seite zeigte eine intensive von dem Ansatz bis zur Spitze gleichmäßige und intensive Goldfärbung.
Die rechte Seite zeigte eine weniger gleichmäßige und weniger intensive Goldfärbung.

### Haltbarkeitsversuch:

Die Kopfhaare wurden beidseitig 12 Tage lang täglich mit einem handelsüblichen Shampoo gewaschen. Das Ergebnis zeigte, daß der Farbabtrag auf der rechten Seite stärker ist als auf der linken Seite.

### Formulierung A3

| | |
|---|---|
| Natrosol® 250 HR | 0,2 |
| Dehyquart® L-80 | 0,3 |
| Panthenol | 0,1 |
| p-Hydroxybenzoesäuremethylester | 0,03 |
| p-Hydroxybenzoesäurepropylester | 0,03 |
| Methoxybutanol | 2,0 |
| HC Red 3 | 0,1 |
| HC Yellow No.2 | 0,2 |
| Wasser | ad 100 |

### Formulierung B3

| | |
|---|---|
| Ammoniumcarbopol-Lösung, 1%ig in Wasser | 17,25 |
| Ammoniumrohagitlösung. 6%ig in Waser | 5,25 |
| Oleth-7 | 5,70 |
| Kaliumoleinseife. 12.5%ig in Wasser | 3,45 |
| Plantaren®2000 | 0,53 |
| Titandioxid Anatas. Typ AS 05 | 0,48 |
| Cetiol® V | 3,45 |
| Cetylalkohol | 16,80 |
| Glycerinmonostearat NSE | 2,85 |
| Phospholipid EFA | 0,85 |
| Tetrasodium EDTA | 0,46 |
| Kieselsäure, hochdisperses, pyrogen | 0,11 |
| p-Toluylendiamin | 0,5 |
| 2-Methylresorcin | 0,2 |
| Resorcin | 0,05 |
| 2-Amino-3-hydroxypyridin | 0,05 |
| m-Aminophenol | 0,02 |
| 4-Chlorresorcin | 0,05 |
| Methoxybutanol | 1,43 |
| Ammoniak, 25%ig im Wasser | ad pH 10,0 |
| Ascorbinsäure | 0,1 |
| Cetyltrimethylammoniumbromid | 0,5 |
| Parfüm | 0,43 |
| Wasser | ad 100,00 |

## Patentansprüche

1. Verfahren zum Färben keratinischer Fasern, **dadurch gekennzeichnet, dass** in einem ersten Schritt ein Vorbehandlungsmittel auf die Fasern aufgetragen wird, das mindestens einen direktziehenden Farbstoff sowie einen faserstrukturverbessernden Wirkstoff enthält, und in einem zweiten Schritt die Fasern einer herkömmlichen Färbung mit mindestens einem synthetischen Farbstoff(vorprodukt) unterzogen werden.

2. Verfahren nach Anspruch 1, **dadurch gekennzeichnet, dass** der direktziehende Farbstoff des Vorbehandlungsmittels ausgewählt ist aus den Nitrobenzolderivaten, den basischen direktziehenden Farbstoffen und den Triphenylmethanderivaten.

3. Verfahren nach Anspruch 1 oder 2, **dadurch gekennzeichnet, dass** der direktziehende Farbstoff ausgewählt ist aus 3-Nitro-4-aminophenol, 4-(3'-Hydroxypropylamino)-3-nitrophenol, 1,4-Bis-(β-hydroxyethyl)-amino-2-nitrobenzol, 1-(β-Hydroxyethylamino)-2-nitro-4-aminobenzol, 1-(β-Hydroxyethylamino)-2-nitrobenzol, 4-(β-Hydroxyethyl-amino)-3-nitrophenol, HC Blue 2, Basic Blue 99, Basic Red 76, Basic Brown 16 und Basic Brown 17.

4. Verfahren nach einem der Ansprüche 1 bis 3, **dadurch gekennzeichnet, dass** das Vorbehandlungsmittel die direktziehenden Farbstoffe in Mengen zwischen 0,001 und 20, bevorzugt zwischen 0,005 und 5, ganz besonders bevorzugt zwischen 0,01 und 1 Gew.-%, bezogen auf das Vorbehandlungsmittel, enthält.

5. Verfahren nach einem der Ansprüche 1 bis 4, **dadurch gekennzeichnet, dass** das Vorbehandlungsmittel als Spray formuliert ist.

6. Verfahren nach einem der Ansprüche 1 bis 6, **dadurch gekennzeichnet, dass** der faserstrukturverbessernde Wirkstoff ausgewählt ist aus der Gruppe, die von Panthenol, physiologisch verträglichen Panthenol-Derivaten, Pflanzenextrakten und Honig gebildet wird.

7. Verfahren nach einem der Ansprüche 1 bis 6, **dadurch gekennzeichnet, dass** der faserstrukturverbessemde Wirkstoff Panthenol ist.

8. Verfahren nach einem der Ansprüche 1 bis 7, **dadurch gekennzeichnet, dass** das Vorbehandlungsmittel zwischen 1 und 30 Minuten, insbesondere zwischen 1 und 10 Minuten, ganz besonders bevorzugt zwischen 3 und 5 Minuten auf dem Haar belassen wird.

9. Verfahren nach einem der Ansprüche 1 bis 8, **dadurch gekennzeichnet, dass** der pH-Wert des Vorbehandlungsmittels zwischen 3 und 9, bevorzugt zwischen 4 und 8, ganz besonders bevorzugt zwischen 4 und 5 liegt.

10. Verfahren nach einem der Ansprüche 1 bis 9, **dadurch gekennzeichnet, dass** die herkömmliche Färbung eine oxidative Färbung ist.

11. Verfahren nach Anspruch 10, **dadurch gekennzeichnet, dass** im zweiten Schritt ein Mittel auf die Fasern aufgetragen wird, das als synthetisches Farbstoffvorprodukt mindestens eine Entwicklerkomponente ausgewählt aus p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol und 2,4,5,6-Tetraaminopyrimidin enthält.

12. Verfahren nach Anspruch 10 oder 11, **dadurch gekennzeichnet, dass** im zweiten Schritt ein Mittel auf die Fasern aufgetragen wird, das mindestens eine Kupplerkomponente, ausgewählt aus 2-Chlor-6-methyl-3-aminophenol, 5-Amino-2-methylphenol, 2-Amino-3-hydroxypyridin, 2,6-Di-(β-hydroxyethylamino)-toluol, 2-Methylresorcin und 1-Naphthol, enthält.

13. Verfahren nach einem der Ansprüche 10 bis 12, **dadurch gekennzeichnet, dass** die oxidative Haarfärbung mit Wasserstoffperoxid erfolgt.

14. Verfahren nach einem der Ansprüche 10 bis 12, **dadurch gekennzeichnet, dass** die oxidative Haarfärbung enzymatisch katalysiert ist.

15. Verwendung eines Vorbehandlungsmittels, enthaltend mindestens einen direktziehenden Farbstoff sowie einen faserstrukturverbessernden Wirkstoff, in Rahmen einer Haarfärbung zur Erzielung einer gleichmäßigen Färbung.

16. Verwendung nach Anspruch 15, **dadurch gekennzeichnet, dass** das Vorbehandlungsmittel als Spray konfektioniert ist.

## Claims

1. Method for colouring keratin fibres, **characterized in that**, in a first step, a pretreatment composition is applied to the fibres which comprises at least one direct dye and an active ingredient which improves the fibre structure, and, in a second step, the fibres are subjected to a conventional colouring with at least one synthetic dye (precursor).

2. Method according to Claim 1, **characterized in that** the direct dye of the pretreatment composition is chosen from the nitrobenzene derivatives, the basic direct dyes and the triphenylmethane derivatives.

3. Method according to Claim 1 or 2, **characterized in that** the direct dye is chosen from 3-nitro-4-aminophenol, 4-(3'-hydroxypropylamino)-3-nitrophenol, 1,4-bis(β-hydroxyethyl)amino-2-nitrobenzene, 1-(β-hydroxyethylamino)-2-nitro-4-aminobenzene, 1-(β-hydroxyethylamino)-2-nitrobenzene, 4-(β-hydroxyethylamino)-3-nitrophenol, HC Blue 2, Basic Blue 99, Basic Red 76, Basic Brown 16 and Basic Brown 17.

4. Method according to one of Claims 1 to 3, **characterized in that** the pretreatment composition comprises the direct dyes in amounts between 0.001 and 20% by weight, preferably between 0.005 and 5% by weight, very particularly preferably between 0.01 and 1% by weight, based on the pretreatment composition.

5. Method according to one of Claims 1 to 4, **characterized in that** the pretreatment composition is formulated as a spray.

6. Method according to one of Claims 1 to 6, **characterized in that** the active ingredient which improves the fibre structure is chosen from the group which is formed by panthenol, physiologically compatible panthenol derivatives, plant extracts and honey.

7. Method according to one of Claims 1 to 6, **characterized in that** the active ingredient which improves the fibre structure is panthenol.

8. Method according to one of Claims 1 to 7, **characterized in that** the pretreatment composition is left on the hair for between 1 and 30 minutes, in particular between 1 and 10 minutes, very particularly preferably between 3 and 5 minutes.

9. Method according to one of Claims 1 to 8, **characterized in that** the pH of the pretreatment composition is between 3 and 9, preferably between 4 and 8, very particularly preferably between 4 and 5.

10. Method according to one of Claims 1 to 9, **characterized in that** the conventional colouring is oxidative colouring.

11. Method according to Claim 10, **characterized in that**, in the second step, a composition is applied to the fibres which comprises, as synthetic dye precursor, at least one developer component chosen from p-phenylenediamine, p-tolylenediamine, p-aminophenol, 1-(2'-hydroxyethyl)-2,5-diaminobenzene, 4-amino-3-methylphenol, 2-aminomethyl-4-aminophenol and 2,4,5,6-tetraaminopyrimidine.

12. Method according to Claim 10 or 11, **characterized in that**, in the second step, a composition is applied to the fibres which comprises at least one coupler component chosen from 2-chloro-6-methyl-3-aminophenol, 5-amino-2-methylphenol, 2-amino-3-hydroxypyridine, 2,6-di(β-hydroxyethylamino)-toluene, 2-methylresorcinol and 1-naphthol.

13. Method according to one of Claims 10 to 12, **characterized in that** the oxidative hair colouring is carried out with hydrogen peroxide.

14. Method according to one of Claims 10 to 12, **characterized in that** the oxidative hair colouring is enzymatically catalysed.

15. Use of a pretreatment composition comprising at least one direct dye and an active ingredient which improves the fibre structure in the course of a hair colouring for achieving an even colour.

16. Use according to Claim 15, **characterized in that** the pretreatment composition is formulated as a spray.

## Revendications

1. Procédé pour la teinture de fibres kératiniques, **caractérisé en ce que**, dans une première étape, on applique un agent de prétraitement sur les fibres, qui contient au moins un colorant montant directement sur les fibres ainsi qu'une substance active améliorant la structure des fibres et, dans une deuxième étape, on soumet les fibres à une teinture usuelle avec au moins un (précurseur de) colorant synthétique.

2. Procédé selon la revendication 1, **caractérisé en ce que** le colorant montant directement sur la fibre de l'agent de prétraitement est choisi parmi les dérivés de nitrobenzène, les colorants basiques montant directement sur la fibre et les dérivés de triphénylméthane.

3. Procédé selon la revendication 1 ou 2, **caractérisé en ce que** le colorant montant directement sur la fibre est choisi parmi le 3-nitro-4-aminophénol, le 4-(3'-hydroxypropylamino)-3-nitrophénol, le 1,4-bis-(β-hydroxyéthyl)-amino-2-nitrobenzène, le 1-(β-hydroxyéthylamino)-2-nitro-4-aminobenzène, le 1-(β-hydroxyéthylamino)-2-nitrobenzène, le 4-(β-hydroxyéthylamino)-3-nitrophénol, le HC Blue 2, le Basic Blue 99, le Basic Red 76, le Basic Brown 16 et le Basic Brown 17.

4. Procédé selon l'une quelconque des revendications 1 à 3, **caractérisé en ce que** l'agent de prétraitement contient les colorants montant directement sur la fibre en des quantités entre 0,001 et 20, de préférence entre 0,005 et 5, de manière tout particulièrement préférée entre 0,01 et 1% en poids par rapport à l'agent de prétraitement.

5. Procédé selon l'une quelconque des revendications 1 à 4, **caractérisé en ce que** l'agent de prétraitement est formulé comme spray.

6. Procédé selon l'une quelconque des revendications 1 à 6, **caractérisé en ce que** la substance active améliorant la structure des fibres est choisie dans le groupe formé par le panthénol, les dérivés du panthénol physiologiquement acceptables, les extraits végétaux et le miel.

7. Procédé selon l'une quelconque des revendications 1 à 6, **caractérisé en ce que** la substance active améliorant la structure des fibres est le panthénol.

8. Procédé selon l'une quelconque des revendications 1 à 7, **caractérisé en ce que** l'agent de prétraitement est laissé sur les cheveux entre 1 et 30 minutes, en particulier entre 1 et 10 minutes, de manière tout particulièrement préférée entre 3 et 5 minutes.

9. Procédé selon l'une quelconque des revendications 1 à 8, **caractérisé en ce que** le pH de l'agent de prétraitement est situé entre 3 et 9, de préférence entre 4 et 8, de manière tout particulièrement préférée entre 4 et 5.

10. Procédé selon l'une quelconque des revendications 1 à 9, **caractérisé en ce que** la teinture usuelle est une teinture d'oxydation.

11. Procédé selon la revendication 10, **caractérisé en ce qu'**on applique dans la deuxième étape un agent sur les fibres, qui contient comme précurseur de colorant synthétique au moins un composant de développement choisi parmi la p-phénylènediamine, la p-toluylènediamine, le p-aminophénol, le 1-(2'-hydroxyéthyl)-2,5-diaminobenzène, le 4-amino-3-méthylphénol, le 2-aminométhyl-4-aminophénol et la 2,4,5,6-tétraaminopyrimidine.

12. Procédé selon la revendication 10 ou 11, **caractérisé en ce que** dans la deuxième étape, on applique un agent sur les fibres qui contient au moins un composant de couplage choisi parmi le 2-chloro-6-méthyl-3-aminophénol, le 5-amino-2-méthylphénol, la 2-amino-3-hydroxypyridine, le 2,6-di-(β-hydroxyéthylamino)-toluène, le 2-méthylrésorcinol et le 1-naphtol.

13. Procédé selon l'une quelconque des revendications 10 à 12, **caractérisé en ce que** la teinture par oxydation des cheveux est réalisée avec du peroxyde d'hydrogène.

14. Procédé selon l'une quelconque des revendications 10 à 12, **caractérisé en ce que** la teinture par oxydation des cheveux est catalysée par voie enzymatique.

15. Utilisation d'un agent de prétraitement, contenant au moins un colorant montant directement sur la fibre ainsi qu'une substance active améliorant la structure des fibres dans le cadre d'une teinture des cheveux en vue d'obtenir une teinture régulière.

16. Utilisation selon la revendication 15, **caractérisée en ce que** l'agent de prétraitement est confectionné sous forme d'un spray.
